(19) Europäisches Patentamt
European Patent Office
Office européen des brevets

(11) **EP 2 613 691 B1**

(12) **EUROPEAN PATENT SPECIFICATION**

(45) Date of publication and mention
of the grant of the patent:
**11.05.2016 Bulletin 2016/19**

(21) Application number: **11773535.7**

(22) Date of filing: **02.09.2011**

(51) Int Cl.:
***A61B 5/0215*** *(2006.01)*

(86) International application number:
**PCT/IB2011/002025**

(87) International publication number:
**WO 2012/032386 (15.03.2012 Gazette 2012/11)**

(54) **METHOD, APPARATUS AND PROGRAM FOR THE AUTOMATIC PROCESSING OF BLOOD PRESSURE SIGNALS**

VERFAHREN, VORRICHTUNG UND PROGRAMM ZUR AUTOMATISCHEN VERARBEITUNG VON BLUTDRUCKSIGNALEN

PROCÉDÉ, APPAREIL ET PROGRAMME POUR LE TRAITEMENT AUTOMATIQUE DE LA PRESSION ARTÉRIELLE À PARTIR DE SIGNAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.09.2010 IT RM20100468**

(43) Date of publication of application:
**17.07.2013 Bulletin 2013/29**

(73) Proprietor: **Romano, Salvatore**
**50144 Firenze (IT)**

(72) Inventor: **Romano, Salvatore**
**50144 Firenze (IT)**

(74) Representative: **Mincone, Antimo**
**Viale Europa 101**
**IT-50126 Firenze (IT)**

(56) References cited:
**US-A- 5 743 267**       **US-A1- 2004 097 813**
**US-A1- 2006 064 021**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a method, an apparatus and a program for the automatic processing of blood pressure that makes it reliable, versatile, effective, simple, and inexpensive, to estimate an energy efficiency of the entire cardiovascular system (RES), providing an estimate of the entropy of the biological system being monitored.

The RES value estimated in accordance with the present invention is useful for monitoring the status and evolution over the time both of the patient seen as a complex biological system as well as parts or components of such a system, i.e. organs or groups of organs of the patient being monitored.

The RES value estimated in accordance with the present invention can also be useful for correctly measure the blood pressure and is self-adapting in relation to the variations of the blood pressure, eliminating the measure alterations of the conventional systems.

The present invention also concerns the respective detection system, as well as means that allow the execution of the method.

It is known that the measure of the blood pressure can be invasive or non-invasive.

Invasive measurement is usually performed through a filling line pressure, linked to an invasive catheter, equipped at an end thereof with a transduction system that converts the measured pressure into a potential difference.

Unlike non-invasive measurement systems, such as the Riva-Rocci cuff, the invasive measure reveals not only the maximum and minimum measured pressure, but also the morphology of the detected signal.

Consequently, the invasive measure, is more reliable than the non-invasive measure, and can reveal the characteristics of the heart-arterial circuit coupling that directly affect the test results. In fact, the systolic and diastolic blood pressure and heart beat morphology are closely related to the contractility of the heart and to the circulatory system, identified by the so-called dynamic impedance $Z_d(t)$, that is linked to the pressure-volume curve (P-V) and is given by the ratio between a pressure value and a time interval.

However, techniques based on invasive blood pressure measuring often give problems due to significant inaccuracies in measures of maximum and minimum pressure and, consequently, also in its morphology. In fact, many authors have demonstrated the existence of an inadequate underdamping of the pressure signal that can lead to significant measurement errors, even several tens of mm Hg.

To solve these problems, some solutions have been proposed based on the application of low-pass filters to the signal pressure (i.e. a frequency processing of the pressure signal that involves the use of a fixed value of the cutoff frequency) and / or the use of mechanical systems able to dampen the frequency components of the detected pressure wave.

In particular, mechanical systems currently used to determine whether the detected signal has or has not a correct underdamping make use of the mechanical square wave test described by RM Gardner in "Direct Blood Pressure Measurement - Dynamic Response Requirements", Anesthesiology, March 1981, Volume 54, Issue 3, ppg 227-236, which requires visual observation of the pressure signal detected by the physician. This is the case, for example, of the systems ROSETM (Resonance Over Shoot Eliminator, available from the U.S. company Becton Dickinson Critical Care System LTD.) and systems Accudynamic ® (available from HOSPIRA - ICU Medical). These systems act mechanically imposing a mechanical damping: in the system Accudynamic ® system, the mechanical damping is adjustable in a small range of damping via a small knob that advances a pin that penetrates into the pressure line in relation to the rotation of the knob; in the ROSES system, the mechanical damping device is set by a micro-bubble and elastic membrane which then acts in a fixed manner for any signal of pressure (prefixed well defined damping).

However, all these systems operate in a pre-fixed (static) on a dynamic problem, since they consider only the frequency spectrum of the signal analyzed by the line pressure. This means that frequency spectra of a same patient in certain conditions are adequate, while in other pathophysiological conditions are clearly inadequate, resulting in overestimation of the blood pressure.

In fact, the correctness of the underdamping is a dynamic problem associated (as well as to the specific patient's cardiovascular system into consideration) to the specific heart rate into account, which can therefore change from beat to beat, so the pressure line responds differently depending on the situation in which it is used.

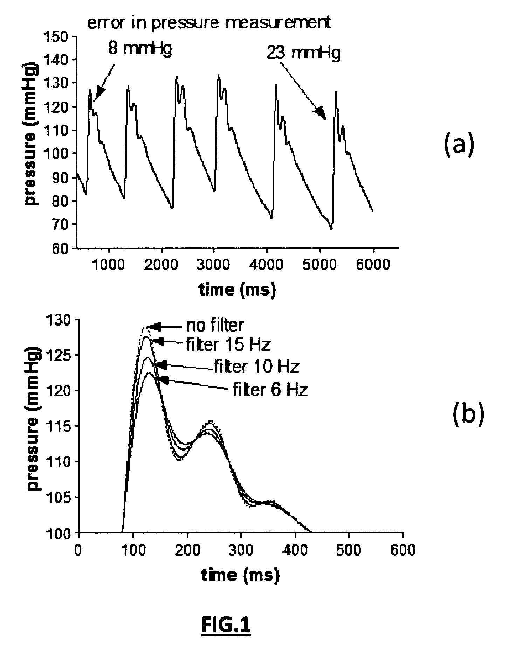

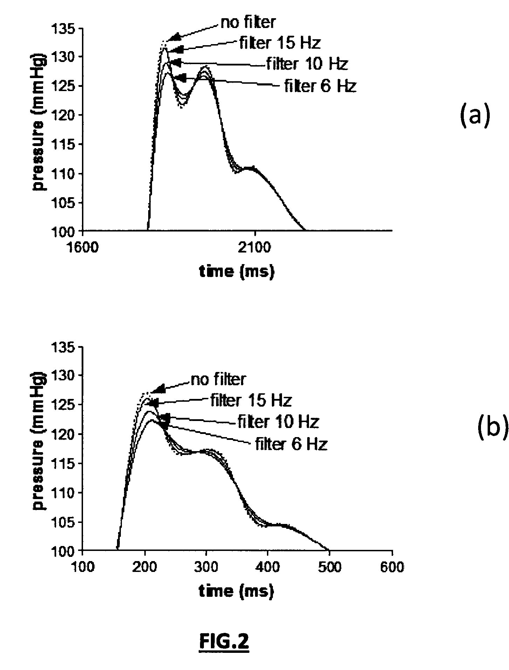

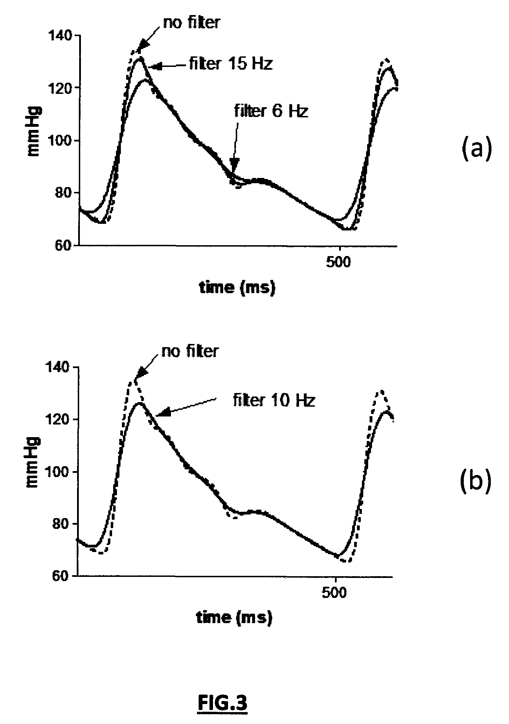

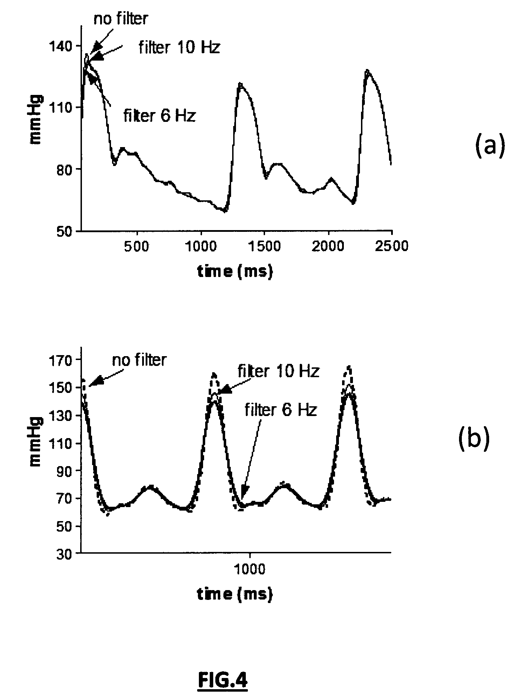

For example, Figure 1 shows a typical blood pressure signal, in which the beats vary in both the morphology and the value of systolic and diastolic pressure (see Fig 1a), and the different result that a conventional system for measuring blood pressure reveals on a specific beat, especially near the systolic pressure, applying no or three different cutoff frequencies (no filter, 15Hz, 10Hz, 6Hz) (see Fig 1b). Figure 2 shows the differences in systolic blood pressure, on two consecutive beats concerning a same blood pressure signal (see Figs. 2a and 2b), obtained by applying no or three different cutoff frequencies (no filter, 15Hz, 10Hz, 6Hz). Figure 3 shows how a conventional measurement system works, in particular near the systolic pressure, in detecting a beat and applying none or two different cutoff frequencies (no filter, 15Hz, 6Hz) (see Fig 3a) and applying none or one cutoff frequency (no filter, 10Hz) (see Fig 3b); in particular, it is evident that the cutoff frequency at 6 Hz causes excessive damping or over-dumping (see Figure 3a), while the filter with cutoff frequency at 10Hz is the most appropriate (see Fig 3b). Figure 4 shows two blood pressure signals in which the same filters work differently: in Figure 4a the filter with cutoff at 10 Hz seems ineffective, while in Figure 4b the same filter with cutoff at 10 Hz acts significantly; in particular, for the beats shown in Figure 4 the filter with cutoff at 6 Hz is the most appropriate.

In addition, the response of the pressure transducer depends not only on the characteristics of length, diameter, material type and liquid filling the line pressure, but also on its coupling with the diameter of the catheter, the arterial tone, the pulse frequency and the stiffness of the vessel of the monitored patient. In this regard, in recent years great efforts have been made to optimize the characteristics of optimal length, diameter, filling liquid, type of material and catheter, in order to limit measurement alterations. In particular, optic pressure lines have been made that can reduce the measurement alterations.

However, all conventional systems do not solve all of the above problems of incorrect measurement of blood pressure, and this is very common especially in cases where detection is most needed, such as for the elder, very young, septic, tachycardic patients and patients that are extremely unstable in both the arterial tone and in the rhythm (eg due to atrial fibrillation).

An object of the present invention is to provide a reliable evaluation of the RES for monitoring the status and evolution over the time both of the patient seen as a complex biological system as well as parts or components of such a system, i.e. organs or groups of organs of the patient being monitored.

Another object of the present invention is to provide a method for correctly measure the blood pressure that is self-adapting in relation to the variations of the blood pressure, eliminating the measure alterations of the conventional systems.

A method and a system according to the present invention are defined by the appended claims.

The method in accordance with the invention involves the determination of a value (RES) characteristic of the functioning of organs and sets of organs of the monitored patient on the basis of the impedance of the direct wave pressure and of the reflected pressure waves obtained through the morphology of the pressure signal in a heartbeat.

The method according to the invention is applicable, for example, also for realizing a system essentially based on a low-pass filter applied to a detected dynamic pressure signal (eg from radial artery, femoral artery, aorta, or pulmonary), in which the filter, that works directly in the time domain, also takes into account the coupling between the pressure line and the characteristic dynamic impedance, obtained from moment to moment analysis of the signal (or curve) of pressure to determine the most appropriate working frequency for the pressure line used. In other words, the method according to the invention is applicable, for example, also to create a system that is based on a characteristic set of conditions on the values of various parameters of the pressure signal, linked to the resulting coupling of the characteristic dynamic impedance of the cardiovascular system with the pressure detection system, instead of a characteristic spectrum of frequencies. In this regard, the pressure signal detected to which the method can be applied according to the invention can also be a recorded signal.

The method according to the invention allows, for example, to determine the proper impedance relating the heart-circulation energy to correct and determine the true pressure and thus, by the relation P-V (pressure-volume), to determine the correct blood flow linked to the corrected pressure waveform and/or to determine the cardiac contractility due to the correct resulting wave pressure. These corrections aiming at obtaining the proper pressure values are valid for detection system based on both filling lines and optic lines, as well as for non-invasive oscillometric piezoelectric detectors (taking into account that all these systems always work on the coupling between the impedance of the detection system and the impedance of the cardiovascular system). In addition, these corrections are valid for pressure signals detected in the arterial central and peripheral system, such as pulmonary artery, aorta, femoral artery, and radial artery.

The method according to the present invention offers many advantages: in relation to the RES values determined in accordance with the invention it is possible to achieve reliable and useful information about the status of organs and sets of organs of the monitored patient; in addition, by measuring the coupling between the measuring line and the patient's cardiovascular system, it is possible to solve the problems due to the coupling of the arterial pressure lines with the dynamic characteristics of the patient whose blood pressure is measured, often varying from beat to beat; moreover, by applying a proper dynamic damping the method according to the invention allows the removal of any alteration from the detected pressure signal, obtaining a correct measurement of the blood pressure and the dynamic impedance, making it possible to obtain, from peripheral pressure, an estimate of the maximum derivative of the pressure inside the left ventricle ($[dP/dt]_{max}$) that has generated the pulse detected in the periphery. In fact, even on the basis of the maximum derivative of the peripheral pressure, the method according to the invention can be used to determine a correction factor (ie, a low-pass filter) that is applied to the said peripheral pressure to estimate the ventricular pressure, taking account of the coupling between the measurement line and the patient's cardiovascular system (for which, for example, in case of a rigid peripheral vessel, it must be applied a large correction). This means that, through the dynamical filtering applied with the method according to the invention, it is removed the contribution related to the stiffness of the artery, leaving the fundamental component linked to the characteristics of the ventricle that has generated the pulse pressure.

In other words, a method according to the present invention allows a proper measurement of blood pressure and, in addition, allows the determination of a correction factor which estimates the maximum derivative dP/dt$_{max}$ of ventricular pressure, determining an energy efficiency of the entire cardiovascular system, and thus providing an estimate of the entropy of the biological system; in fact, through the concept of efficiency it is possible to take into account the "unrecoverable" mechanical energy associated, for example, to a cardiac cycle. This efficiency describes how much of the "reserves" of the biological system of the organism into account is consumed, since consumption of reserves means consuming the "components" of the physiological system (eg, organs, glands (bio-chemical reactions), heart's electrical system, etc..).

The present invention will now be described, for illustrative and not limitative purposes, with reference to preferred embodiments, with particular reference to the figures of the annexed drawings, which relate to the application of the method for the measurement of blood pressure, wherein:

Figure 1 shows a blood pressure signal (Fig. 1a) and the different result obtained by a conventional blood pressure measurement by applying on a specific beat none or three different cutoff frequencies (see Fig 1b);
Figure 2 shows two consecutive beats in the same blood pressure signal obtained by applying a conventional system with no or three different cutoff frequencies;
Figure 3 shows a pulse obtained by applying a conventional system with none or two different first cutoff frequencies (Fig. 3a) and applying a conventional system with none or a second cutoff frequency (Fig. 3b);
Figure 4 shows two signals of blood pressure obtained applying, with a conventional system, none or two identical filters;
Figure 5 shows a block diagram of a preferred embodiment of the automated method according to the invention, and Figure 6 shows a pressure signal of a single pulse to which is applied the method of Figure 5.

The inventor has developed a method for estimating the RES, which also allows to correctly measure blood pressure from a detected pressure signal, that operates in the time domain to discriminate if the detected signal is an appropriate measure or not, and if it is not, the analysis in the time domain automatically selects a low pass filter to apply to have the correct blood pressure values and waveform. In this regard, the inventor has verified that the method according to the invention is applicable also to provide a pressure signal having an adequate underdamping by means of the square wave test before and after applying the filter in accordance with the method of the present invention.

Preferably, the detected pressure signal is made available through invasive detection technique, e.g. a so-called filling pressure line or an optical-fiber pressure line in femoral, radial, brachial or pulmunary artery or aorta by non-invasive detection technique, e.g. piezoelectric or oscillometric plethysmography. However, the detected pressure signal to which is applied the method according to the invention can also be a signal recorded and subsequently analyzed by subjecting it to the method of the invention, whose scope of protection, therefore, does not include any invasive surgical procedure on the patient's body.

More specifically, the method according to the invention is based on the pulse frequency (i.e. it makes use of the total time interval of the heartbeat and the relative distances of individual points of pressure within the heartbeat itself), some characteristic points of the heartbeat using the first derivative of the measured blood pressure (dP/dt) and the second derivative of the measured blood pressure (d$^2$P/dt$^2$), and some dynamic impedance values Zd (t) at specific moments of the direct wave pressure (which propagates from the heart to the periphery) and of the reflected pressure waves (which propagate from the periphery to the heart).

From the values of the dynamic impedance thus obtained, the method involves to evaluate whether the pressure is correct, and, if not, then it involves to select a cutoff frequency, preferably between 0.5 Hz and 100 Hz, more preferably between 2 Hz and 80 Hz, even more preferably between 3 Hz and 60 Hz of the low-pass filter to apply to the detected pressure signal, such that cutoff frequency be the most appropriate detection conditions, so that the method dynamically adapts to the variations of the detection that can occur from beat to beat and from instant to instant.

In other words, the method according to the invention allows to exploit the unique characteristics of pulsating heart beat into account, and through them determines an appropriate low-pass filter with variable cutoff frequency in order to apply a suitable underdamping.

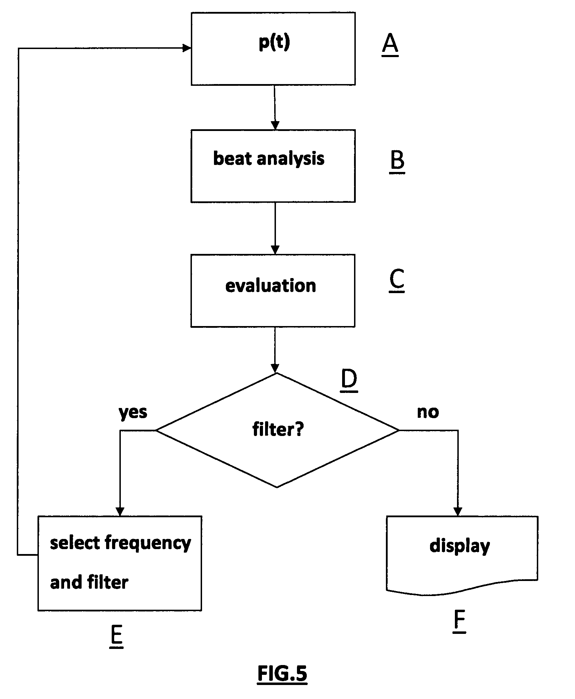

With reference to Figure 5, a possible form of implementation and application of the method according to the invention comprises the following steps:

A. having a pressure signal detected by means of a pressure transducer (preferably by an invasive arterial pressure line, or through non-invasive technique, eg by means of plethysmographic oscillometric method), which is sampled, preferably with a sampling frequency of 1000 Hz;
B. automatically analyze and discriminate the morphology of the sampled wave (i.e. the signal) pressure for each heartbeat (ranging from a starting point of diastolic blood pressure to a next point of diastolic pressure, considering the starting point of the beat, i.e. the starting point of the diastolic pressure, as the point of diastolic pressure of the beat);

C. for each heartbeat, to determine evaluation values consisting of (or including) a direct pressure wave impedance and an impedance of the reflected pressure waves and a consequent energy efficiency of the entire cardiovascular system;

D. to verify whether it is necessary to apply a low-pass filter and, in the positive case, perform the step E, otherwise perform the step F assuming that the measured pressure signal is identical to sampled pressure signal;

E. to select the cutoff frequency of the low-pass filter, on the basis of the analysis made at step B and the evaluation made at step C, and apply a low-pass filter to the sampled pressure signal, thus obtaining a new sampled pressure signal, and returning to step B;

F. providing the measured pressure signal, preferably by displaying it on a display.

The step B for the automatic analysis of the morphology of the detected pressure wave (i.e. the signal) during a heartbeat discriminates and analyzes the shape or morphology of a heart beat detecting both characteristics of pressure and times (which, as will be disclosed below, are considered intervals starting from the beginning of the heart beat - that is, the initial diastolic pressure moment - or, in reverse, from the moment of the end of the beat) relating to specific points starting from the beginning of the heartbeat, in particular the characteristic points of diastolic blood pressure (initial in the beat), systolic pressure, dicrotic, and resonance in a single heartbeat.

In more detail, the step B includes the following sub-steps:

B.1 determining the pressure and the instant of the point of diastolic pressure (corresponding to an "initial" absolute minimum pressure signal in the single heartbeat), the systolic pressure point (corresponding to the absolute maximum of the pressure signal in the single heart beat), and the dicrotic point (corresponding to the point where the aortic heart valve is closed and that mathematically corresponds to a relative maximum of the second derivative or a relative minimum of the pressure curve that occurs immediately after the point of systolic pressure),

B.2 determining the total number $N_{dP-max}$ of points of relative maximum (including the absolute maximum) of the first derivative dP/dt of the pressure signal (sampled) in the range of a single heartbeat;

B.3 determining the points of relative maximum (including the absolute maximum) of the second derivative $d^2P/dt^2$ of the pressure signal (sampled) in the range of a single heartbeat, and

B.4 determining the $N_{dP-max}$ points of relative maximum of the second derivative $d^2P/dt^2$ having the highest values (i.e. selecting a number of points of relative maximum of the second derivative $d^2P/dt^2$ equal to the total number $N_{dPmax}$ of points of relative maximum of the first derivative dP/dt previously determined) and determining the $N_{dP-max}$ time instants $t_{d2P-max}(i)$ (with i varying from 1 to $N_{dP-max}$) in which they occur, considering the point of the pressure signal in said $N_{dP-max}$ time instants $t_{d2P-max}(i)$ as resonance points.

In particular, the connection between the number $N_{dP-max}$ of relative maximum of the second derivative $d^2P/dt^2$ and the total number of points of relative maximum of the first derivative dP/dt in the range of a single heartbeat allows the elimination of the relative maximum points of the second derivative $d^2P/dt^2$ due to noise. In this regard, the point of diastolic peak (i.e. the highest relative maximum after the dicrotic notch - i.e. the dicrotic point - and after the bump following the dicrotic point) is always selected in step B.4 among the resonance points.

As a not limitative example, the heartbeat and the corresponding pressure characteristic points can be discriminated and individuated by means of a method as that disclosed in WO 2004/084088.

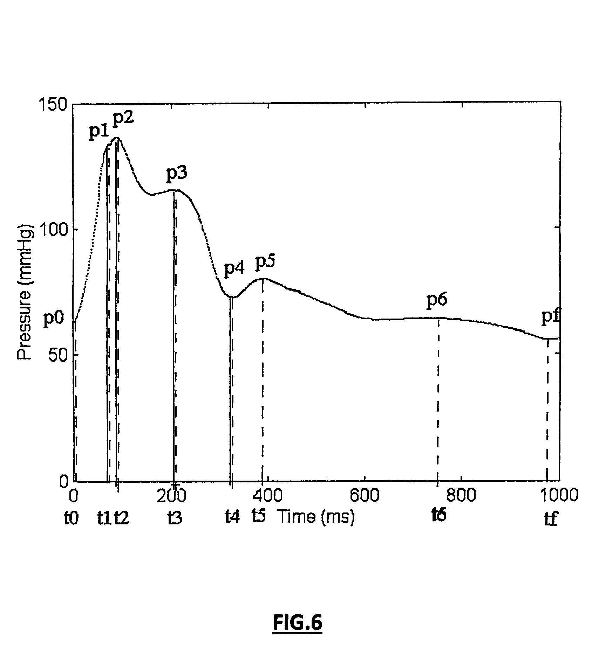

By way of example, Figure 6 shows the pressure signal of a single heartbeat in which:

- p0 is the value of diastolic blood pressure (initial in the beat), at the instant to of beat starting (i.e. time of the point of initial diastolic pressure in the beat);
- p2 is the value of systolic pressure, at time t2 of the systolic pressure point;
- p4 is the pressure at the dicrotic point, which occurs at time t4;
- p1, p3, p5, p6, and pf are the pressure values at the point of resonance occurring at times t1, t3, t5, t6 and tf (determined on the basis of the total number $N_{dP\_max}$ of points of relative maximum of the first derivative dP/dt, amounting to 5, and the selection of the corresponding 5 points - with higher value - of relative maximum of the second derivative $d^2P/dt^2$).

In Figure 6, the point p5 is the hump that follows the dicrotic point, while the point p6 is the diastolic peak (the relative maximum after the dicrotic notch and the possible bump immediately after this).

Step C determines an estimated value of an energy efficiency of the entire cardiovascular system, providing an estimate of the entropy of the biological system. In particular, it is determined - and preferably displayed - a value, here defined as Resulting of the Energy ratio of the System or RES, of the cardiovascular system obtained on the basis of the direct pressure wave impedance and the reflected pressure waves obtained by the morphology of the pressure signal in a heartbeat. Said impedances are determined considering pressure and time values of characteristic points of the heartbeat, including not only the diastolic pressure points (initial point of the beat), systolic pressure, and dicrot (situated in the time range comprising the sub-ranges diastolic-systolic and systolic-dicrotic - that is, in the systolic phase of the single heartbeat), but also the resonance points individuated during a single heartbeat in the step B (precisely, in the sub-step B.4) disclosed above, between said resonance points being always present a diastolic peak (.i.e. peak after the dicrotic notch in the diastolic phase of the single heartbeat).

In more detail, the step C includes the following sub-steps:

C.1 determining the direct pressure wave impedance $Z_D$ on the basis of a sum of dynamic impedances of a first set of points in the heartbeat (set_1) comprising those points among the characteristic points disclosed above belonging to the systolic phase of the single heartbeat (ie in the range from the point of initial diastolic pressure to the dicrotic point), with the exception of the initial diastolic point;

C.2 the impedance $Z_R$ of the reflected pressure waves is determined on the basis of a sum of dynamic impedances of a second set of points of the heartbeat (set_2) including all the characteristic points mentioned above (that belong to the entire heartbeat);

C.3 determining the RES value as the ratio between the impedance $Z_D$ of the direct wave pressure and the impedance $Z_R$ of the reflected waves.

In particular, in relation to the impedance $Z_D$ of the direct wave pressure, for each point of the first set it is determined a corresponding dynamic direct impedance $Z_{d-D}(t)$, given by the ratio between the pressure value relating to that point and the distance of the respective time from the initial time of the beat, that is, from the instant of the initial diastolic point (this why in the first set of points it is excluded the point of initial diastolic, whose dynamic impedance would have at the denominator the value 0). The value of the impedance $Z_D$ of the direct wave pressure is obtained by adding with alternating signs the dynamic impedances of the first set, ordered according their temporal order starting from the instant of the initial diastolic pressure to the dicrotic instant, giving a positive sign to the dynamic impedance of the first point of the first set.

Similarly, in relation to the impedance $Z_R$ of the reflected waves of pressure, for each point of the second set it is also determined a corresponding dynamic reflected impedance $Z_{d\_R}(t)$ that is given by the ratio between the pressure at that point and the distance of the respective instant from the instant of the final beat. The value of the impedance $Z_R$ of reflected waves of pressure is obtained by adding the dynamic impedances with alternating signs of the second set of points thus determined, sorted according to their reverse temporal order starting from the instant of the final beat to the instant of the initial diastolic pressure, giving a positive sign to the dynamic impedance of the first point of the second set.

In other words, the impedances $Z_D$ and $Z_R$ of the direct wave and of the reflected waves of pressure are given each by a respective series of terms (ie their direct dynamic impedances $Z_{d\_D}(t)$ and reflected impedances $Z_{d\_R}(t)$) oscillating (because they are considered with alternating signs) whose value becomes progressively smaller (because the value in the denominator of the dynamic impedance increases progressively).

As said above, the RES value is determined by the ratio between the impedance of the direct pressure wave $Z_D$ (determined on the basis of the first set of points) and the impedance $Z_R$ of the reflected pressure waves (determined on the basis of the second set of points):

$$RES = Z_D / Z_R.$$

This value of the RES represents an energy efficiency for obtaining a given homeostasis of the entire cardiovascular-respiratory system.

In the graph given by way of not limitative example of Figure 6, the points belonging to the first set (set_1) are indicated by continuous vertical lines (from the axis of time to the value of pressure corresponding to the respective point) and the points belonging to the second set (set-2) are indicated with dashed vertical lines, so that the points belonging to both the first and the second set are denoted by a pair of vertical lines (one continuous and dashed the other). As shown, the first set includes (in order starting from the instant of initial diastolic pressure to the dicrotic) the points indicated by p1, p2, p3, and p4, while the second set includes (in the temporal reverse order starting from the end of the beat up at the instant of the initial diastolic pressure) the points indicated with pf, p6, p5, p4, p3, p2, p1, p0.

The value of the impedance of the direct pressure wave $Z_D$, for the heartbeat shown in Figure 6, is

$$Z_D = \frac{p1}{t1} - \frac{p2}{t2} + \frac{p3}{t3} - \frac{p4}{t4}$$

while the value of the impedance $Z_R$ of the reflected pressure waves, assuming that the period of the single heartbeat shown the Figure is T, is

$$Z_R = \frac{pf}{(T-tf)} - \frac{p6}{(T-t6)} + \frac{p5}{(T-t5)} - \frac{p4}{(T-t4)} + \frac{p3}{(T-t3)} - \frac{p2}{(T-t2)} + \frac{p1}{(T-t1)} - \frac{p0}{(T-t0)}$$

The verification step D makes use, as mentioned before, of a characteristic set of conditions concerning the values obtained in steps B and C to determine whether the single heartbeat is affected by underdumping, i.e. whether the systolic pressure is overestimated or the diastolic pressure is underestimated, or if, on the contrary, the morphology of the heartbeat is correct. If by this evaluation it is found that the heartbeat is within the limits given by said characteristic set of conditions, then no frequency filtering is applied and the method implies the restitution (in step F) of a measured pressure signal that corresponds to the pressure sampled signal unchanged as regards frequencies and amplitudes thereof. If, on the contrary, the characteristics of the heartbeat being monitored are within intervals defined by the said characteristic set of conditions, step E provides a correction by modifying the spectrum of the sampled pressure signal by applying a low pass filter with a determined cutoff frequency, and steps B, that analyzes the thus filtered sampled pressure signal, and C, determining evaluation values, are re-run and in a further phase D it is checked if the values thus obtained are or not within the limits imposed from said characteristic set of conditions. In other words, the sampled and filtered pressure signal of the heartbeat is once again analyzed: if the values obtained are in agreement with the intervals defined by the characteristic set of conditions, then the method returns (in the step F) a measured pressure signal corresponding to the sampled pressure signal obtained through the last filtering (without applying additional filtering); but if the values obtained are not within the limits imposed by the characteristic set of conditions, then there is the repetition of the filtering with a cutoff frequency suitably selected, and the method executes iteratively the step B until it is obtained a signal whose values are in agreement with the intervals defined by the characteristic set of conditions.

In more detail, the step D verifies if, for the value of RES determined in step C, the values of the first derivative dP/dT of the pressure signal and the values of the second derivative $d^2P/dt^2$ of the pressure signal in the entire heartbeat are lower than respective maximum threshold values $T_d$ and $T_{d2}$ (function of the RES value), and in this case it is not necessary to apply any filter to the pressure signal and the method involves to directly execute the step F, otherwise the method involves the execution of the step E, applying a low-pass filter to the pressure signal with a determined cutoff frequency, and the execution of all the steps starting from step B.

In particular, the possible values of RES are subdivided into three or more, preferably pre-four, adjacent intervals of variability, and the values $T_d$ and $T_{d2}$ are a function of the interval to which the RES determined in step C belongs. Preferably:

-   if the value of RES is not less (or even more) of a minimum threshold $T_{RES\_min}$ not less than 0.3, preferably not less than 0.4, more preferably not less than 0.5,

    -   the maximum threshold value $T_d$ of the first derivative dP/dt of the pressure signal is not greater than 1.2 mmH/ms, preferably not greater than 1.1 mmH/ms, more preferably not more than 1.0 mmH/ms, and
    -   the maximum threshold value $T_{d2}$ of the second derivative $d_2P/dt^2$ of the pressure signal is not greater than 0.2 mmH/ms$^2$, preferably not more than 0.17 mmH/ms$^2$, more preferably not more than 0.15 mmH/ms$^2$,
    -   if the value of RES is variable within a first interval (mathematically open or closed) whose lower limit is greater than 0 and whose upper limit is not greater than the minimum threshold $T_{RES\_min}$, the first interval being preferably from 0, 3 to 0.5,
    -   the maximum threshold value $T_d$ of the first derivative dP/dt of the pressure signal is not greater than 1.6 mmH/ms, preferably not more than 1.4 mmH/ms, more preferably not more than 1.2 mmH/ms, and
    -   the maximum threshold value $T_{d2}$ of the second derivative $d_2P/dt^2$ of the pressure signal is not greater than 0.25 mmH/ms$^2$, preferably not greater than 0.22 mmH/ms$^2$, more preferably not more than 0.20 mmH/ms$^2$,

-   if the RES value is variable within a second range (mathematically open or closed) contiguous to and preceding the first interval (in the sense that the lower limit of the first interval coincides with the upper limit of the second interval), whose lower limit is not less than 0, preferably 0,

    -   the maximum threshold value $T_d$ of the first derivative dP/dt of the pressure signal is not greater than 1.6 mmH/ms, preferably not more than 1.4 mmH/ms, more preferably not more than 1.2 mmH/ms, and

- the maximum threshold value $T_{d2}$ of the second derivative $d_2P/dt^2$ of the pressure signal is not greater than 0.35 mmH/ms$^2$, preferably not greater than 0.30 mmH/ms$^2$, more preferably not more than 0.27mmH/ms$^2$, and yet more preferably not more than 0.25 mmH/ms$^2$,

- if the RES value is lower (or even not greater) than a maximum threshold value $T_{RES-max}$ coinciding with the lower limit of the second interval,

  - the maximum threshold value $T_d$ of the first derivative $dP/dt$ of the pressure signal is not greater than 2.0 mmH/ms, preferably not more than 1.8 mmH/ms, more preferably not more than 1.6 mmH/ms, and
  - the maximum threshold value $T_{d2}$ of the second derivative $d_2P/dt^2$ of the pressure signal is not greater than 0.45 mmH/ms$^2$, preferably not greater than 0.40 mmH/ms$^2$, more preferably not more than 0.37mmH/ms$^2$, and yet more preferably not more than 0.3 mmH/ms$^2$.

The verification step D tests that there is no need to apply any filter to the pressure signal when any of the following four sets of conditions is satisfied:

- $RES \geq 0.5$, the first derivative $dP/dt$ is less than 1.0 mmH/ms throughout the heartbeat, and the second derivative $d_2P/dt^2$ is less than 0.15 mmH/ms$^2$ throughout the heartbeat;
- $0.3 \leq RES < 0.5$, the first derivative $dP/dt$ is less than 1.2 mmH/ms throughout the heartbeat, and the second derivative $d_2P/dt^2$ is less than 0.2 mmH/ms$^2$ throughout the heartbeat;
- $0.0 \leq RES < 0.3$, the first derivative $dP/dt$ is less than 1.2 mmH/ms throughout the heartbeat, and the second derivative $d_2P/dt^2$ is less than 0.25 mmH/ms$^2$ throughout the heartbeat;
- $RES < 0.0$, the first derivative $dP/dt$ is less than 1.6 mmH/ms throughout the heartbeat, and the second derivative $d_2P/dt^2$ is less than 0.35 mmH/ms$^2$ throughout the heartbeat.

As mentioned above, the step E selects the cutoff frequency of the low-pass filter, based on the analysis of step B and of the determination of step C, and applies the low-pass filter to the sampled pressure signal. In particular, the step E selects the cutoff frequency of the lowpass filter based on the value of RES and values of the first derivative and second derivative values of the pressure signal in the whole heart rate, as follows: RES values are discriminated in three or more, preferably four, adjacent intervals of variability (preferably corresponding to those used in the verification step D), for each of them the values of first derivative $dP/dt$ of the pressure signal are discriminated in three or more, preferably six, adjacent intervals of variability, and, for at least one of the ranges of values of the first derivative $dP/dt$, the values of the second derivative $d_2P/dt^2$ of the pressure signal are discriminated into three or more, preferably four, not superimposed variability intervals (adjacent to each other and, where applicable, adjacent to the range of values of the second derivative $d_2P/dt^2$ for which according the method no low-pass filter is applied), thus selecting a corresponding cut-off frequency of the low pass filter to apply.

Step E discriminates the RES values in four adjacent variability intervals (corresponding to those of the verification step D), for each of them discriminates the values of the first derivative $dP/dT$ of the pressure signal in six adjacent variability intervals, and for the first interval of values of the first derivative $dP/dT$ discriminates the values of the second derivative $d_2P/dt^2$ in four adjacent variability intervals (that are consecutive to the interval corresponding to the set of conditions for which no low-pass filter is applied). In more detail, according to a preferred embodiment, the cutoff frequency of the filter is determined as follows:

  1. if the RES value meets the condition $RES \geq 0.5$

    1.1 if the values of the first derivative throughout the heartbeat satisfy the condition

$$dP/dt < 1.0 \text{ mmH/ms}$$

    1.1.1 if the values of the second derivative throughout the heartbeat satisfy the condition

$$0.15 \text{ mmH/ms} \leq d^2P/dt^2 < 0.25 \text{ mmH/ms}$$

    it is applied a lowpass filter with cutoff frequency of 15 Hz;
    1.1.2 if the values of the second derivative throughout the heartbeat satisfy the condition

$$0.25 \text{ mmH/ms} \le d_2P/dt^2 < 0.30 \text{ mmH/ms}$$

it is applied a lowpass filter with cutoff frequency of 12 Hz;

1.1.3 if the values of the second derivative throughout the heartbeat satisfy the condition

$$0.30 \text{ mmH /ms} \le d_2P/dt^2 < 0.35 \text{ mmH/ms}$$

it is applied a lowpass filter with cutoff frequency of 8 Hz;

1.1.4 if the values of the second derivative throughout the heartbeat satisfy the condition

$$d_2P/dt^2 \ge 0.35 \text{ mmH / ms}$$

it is applied a lowpass filter with cutoff frequency of 7 Hz;

1.2 if the values of the first derivative throughout the heartbeat satisfy the condition

$$\text{mmH/ms} \le dP/dt < 1.3 \text{ mmH/ ms}$$

it is applied a lowpass filter with cutoff frequency of 12 Hz;

1.3 if the values of the first derivative throughout the heartbeat satisfy the condition

$$1.3 \text{ mmH/ms} \le dP/dt < 1.5 \text{ mmH/ms}$$

it is applied a lowpass filter with cutoff frequency of 8 Hz;

1.4 if the values of the first derivative throughout the heartbeat satisfy the condition

$$\text{mmH/ms} \le dP /dt < 2.5 \text{ mmH/ms}$$

it is applied a lowpass filter with cutoff frequency of 7 Hz;

1.5 if the values of the first derivative throughout the heartbeat satisfy the condition

$$\text{mmH/ms} \le dP/dt < 3.0 \text{ mmH/ms}$$

it is applied a lowpass filter with cutoff frequency of 6 Hz;

1.6 if the values of the first derivative throughout the heartbeat satisfy the condition

$$dP/dt \ge 3.0 \text{ mmH/ms}$$

it is applied a lowpass filter with cutoff frequency of 3 Hz;

2. if the RES value meets the condition $0.3 \le RES < 0.5$

2.1 if the values of the first derivative throughout the heartbeat satisfy the condition

$$dP/dt < 1.2 \text{ mmH/ms}$$

2.1.1 if the values of the second derivative throughout the heartbeat satisfy the condition

$$0.2 \text{ mmH/ms} \leq d^2P/dt^2 < 0.25 \text{ mmH/ms}$$

it is applied a lowpass filter with cutoff frequency of 15 Hz;
2.1.2 if the values of the second derivative throughout the heartbeat satisfy the condition

$$0.25 \text{ mmH/ms} \leq d_2P/dt^2 < 0.35 \text{ mmH/ms}$$

it is applied a lowpass filter with cutoff frequency of 12 Hz;
2.1.3 if the values of the second derivative throughout the heartbeat satisfy the condition

$$0.35 \text{ mmH /ms} \leq d_2P/dt^2 < 0.45 \text{ mmH/ms}$$

it is applied a lowpass filter with cutoff frequency of 8 Hz;
2.1.4 if the values of the second derivative throughout the heartbeat satisfy the condition

$$d_2P/dt^2 \geq 0.45 \text{ mmH / ms}$$

it is applied a lowpass filter with cutoff frequency of 7 Hz;

2.2 if the values of the first derivative throughout the heartbeat satisfy the condition

$$1.2 \text{ mmH/ms} \leq dP/dt < 1.5 \text{ mmH/ ms}$$

it is applied a lowpass filter with cutoff frequency of 13 Hz;
2.3 if the values of the first derivative throughout the heartbeat satisfy the condition

$$1.5 \text{ mmH/ms} \leq dP/dt < 1.8 \text{ mmH/ms}$$

it is applied a lowpass filter with cutoff frequency of 10 Hz;
2.4 if the values of the first derivative throughout the heartbeat satisfy the condition

$$1.8 \text{ mmH/ms} \leq dP /dt < 2.5 \text{ mmH/ms}$$

it is applied a lowpass filter with cutoff frequency of 8 Hz;
2.5 if the values of the first derivative throughout the heartbeat satisfy the condition

$$2.5 \text{ mmH/ms} \leq dP/dt < 3.5 \text{ mmH/ms}$$

it is applied a lowpass filter with cutoff frequency of 6 Hz;
2.6 if the values of the first derivative throughout the heartbeat satisfy the condition

$$dP/dt \geq 3.5 \text{ mmH/ms}$$

it is applied a lowpass filter with cutoff frequency of 3 Hz;

3. if the RES value meets the condition $0.0 \leq RES < 0.3$

3.1 if the values of the first derivative throughout the heartbeat satisfy the condition

$$dP/dt < 1.2 \text{ mmH/ms}$$

3.1.1 if the values of the second derivative throughout the heartbeat satisfy the condition

$$0.25 \text{ mmH/ms} \leq d^2P/dt^2 < 0.30 \text{ mmH/ms}$$

it is applied a lowpass filter with cutoff frequency of 15 Hz;
3.1.2 if the values of the second derivative throughout the heartbeat satisfy the condition

$$0.30 \text{ mmH/ms} \leq d_2P/dt^2 < 0.40 \text{ mmH/ms}$$

it is applied a lowpass filter with cutoff frequency of 12 Hz;
3.1.3 if the values of the second derivative throughout the heartbeat satisfy the condition

$$0.40 \text{ mmH /ms} \leq d_2P/dt^2 < 0.50 \text{ mmH/ms}$$

it is applied a lowpass filter with cutoff frequency of 8 Hz;
3.1.4 if the values of the second derivative throughout the heartbeat satisfy the condition

$$d_2P/dt^2 \geq 0.50 \text{ mmH / ms}$$

it is applied a lowpass filter with cutoff frequency of 5 Hz;

3.2 if the values of the first derivative throughout the heartbeat satisfy the condition

$$1.2 \text{ mmH/ms} \leq dP/dt < 1.5 \text{ mmH/ ms}$$

it is applied a lowpass filter with cutoff frequency of 13 Hz;
3.3 if the values of the first derivative throughout the heartbeat satisfy the condition

$$1.5 \text{ mmH/ms} \leq dP/dt < 1.8 \text{ mmH/ms}$$

it is applied a lowpass filter with cutoff frequency of 10 Hz;
3.4 if the values of the first derivative throughout the heartbeat satisfy the condition

$$1.8 \text{ mmH/ms} \leq dP /dt < 2.5 \text{ mmH/ms}$$

it is applied a lowpass filter with cutoff frequency of 8 Hz;
3.5 if the values of the first derivative throughout the heartbeat satisfy the condition

$$2.5 \text{ mmH/ms} \leq dP/dt < 3.5 \text{ mmH/ms}$$

it is applied a lowpass filter with cutoff frequency of 6 Hz;
3.6 if the values of the first derivative throughout the heartbeat satisfy the condition

$$dP/dt \geq 3.5 \text{ mmH/ms}$$

it is applied a lowpass filter with cutoff frequency of 3 Hz;

4. if the RES value meets the condition RES <0.0

4.1 if the values of the first derivative throughout the heartbeat satisfy the condition

$$dP/dt < 1.6 \text{ mmH/ms}$$

4.1.1 if the values of the second derivative throughout the heartbeat satisfy the condition

$$0.35 \text{ mmH/ms} \leq d^2P/dt^2 < 0.40 \text{ mmH/ms}$$

it is applied a lowpass filter with cutoff frequency of 15 Hz;
4.1.2 if the values of the second derivative throughout the heartbeat satisfy the condition

$$0.40 \text{ mmH/ms} \leq d_2P/dt^2 < 0.45 \text{ mmH/ms}$$

it is applied a lowpass filter with cutoff frequency of 12 Hz;
4.1.3 if the values of the second derivative throughout the heartbeat satisfy the condition

$$0.45 \text{ mmH /ms} \leq d_2P/dt^2 < 0.50 \text{ mmH/ms}$$

it is applied a lowpass filter with cutoff frequency of 8 Hz;
4.1.4 if the values of the second derivative throughout the heartbeat satisfy the condition

$$d_2P/dt^2 \geq 0.50 \text{ mmH / ms}$$

it is applied a lowpass filter with cutoff frequency of 10 Hz;

4.2 if the values of the first derivative throughout the heartbeat satisfy the condition

$$1.6 \text{ mmH/ms} \leq dP/dt < 1.8 \text{ mmH/ ms}$$

it is applied a lowpass filter with cutoff frequency of 13 Hz;
4.3 if the values of the first derivative throughout the heartbeat satisfy the condition

$$1.8 \text{ mmH/ms} \leq dP/dt < 2.0 \text{ mmH/ms}$$

it is applied a lowpass filter with cutoff frequency of 10 Hz;
4.4 if the values of the first derivative throughout the heartbeat satisfy the condition

$$2.0 \text{ mmH/ms} \leq dP /dt < 2.4 \text{ mmH/ms}$$

it is applied a lowpass filter with cutoff frequency of 8 Hz;
4.5 if the values of the first derivative throughout the heartbeat satisfy the condition

$$2.4 \text{ mmH/ms} \leq dP/dt < 3.2 \text{ mmH/ms}$$

it is applied a lowpass filter with cutoff frequency of 6 Hz;
4.6 if the values of the first derivative throughout the heartbeat satisfy the condition

$$dP/dt \geq 3.2 \ mmH/ms$$

it is applied a lowpass filter with cutoff frequency of 3 Hz.

The values indicated above for lower and/or upper limits of the various adjacent intervals for the RES, the various intervals of the first derivative dP/dt and the various intervals of second derivative $d_2P/dt^2$, and the values selected for the cutoff frequencies, are only indicative and not exhaustive, and may be increased or decreased preferably not more than 25%, more preferably no more than 20%, still more preferably no more than 15%, even more preferably no more than 10%.

The inventor has checked through an analysis in the frequency domain of the frequency spectra of the pressure signal sampled in the heartbeat and its first and second derivatives in the frequency domain, the effectiveness of low-pass filtering according to the method of the present invention.

Finally, the step F provides for displaying the sampled pressure signal, eventually obtained through the last filtering, on a display, so as to show the measure and morphology of the pressure signal thus obtained.

The detailed description provided above is referred, as mentioned before, to the application of the method for determining the RES in order to provide a correct measure of the blood pressure.

However, as mentioned before, the determination of the RES is also useful for other purposes. For example, in a young patient with normal pressure and heart rate, it is RES≈0.3. If RES> 0.3 (for example, RES=0.5 to 0.6), there is an indication of an extreme vasodilaton of the patient due to a probable septic shock and consequently poor kidney perfusion. A negative value of the RES (eg -0.5 or -0.6) would be attributable to poor cardiac contractility in relation to the rigidity of the vessels of the cardiovascular system. Or, rapid increases in the RES in transition from spontaneous breathing to apnea are indicative of submassive pulmonary embolism. Still, in the cardiac monitoring in the intensive care, through the determination of the RES it is possible to control the efficiency of a counter-pulser (IABP): in fact, beats properly counter-pulsed give rise to RES values much higher than not counter-pulsed or not properly counter-pulsed beats. In addition, during the extracorporeal circulation, the RES assumes very negative values becoming progressively more negative with the prolongation of this state. Therefore, the RES can highlight the effectiveness of mechanical support mechanisms of the heart and lung functions of patients to which these mechanisms are applied.

The said values of RES can vary depending on the point where the pressure signal is taken (radial artery, femoral artery, aortic arch, pulmonary artery). Signals taken at the radial artery produce values of the RES higher than those determined on the basis of signals taken in larger arteries. The evolution of the RES over time, ie during the period of monitoring of the patient, provides indications about the response into of the biological system to various stimuli and stress, including pharmacological stress and stimuli.

From the above description it is evident that the determination of RES according to the invention can have several practical applications. The applicative examples given above are not to be interpreted in any limitative sense.

The above description illustrates some embodiments and variants of the present invention but it is meant that anyone skilled in the art may make modifications and changes without, however, departing from the protection granted as defined by the appended claims.

## Claims

1. Method for the automatic processing of blood pressure signals comprising at least the following steps:

   A. having a detected pressure signal P(t) sampled for one or more heartbeats, each heartbeat starting at an initial instant coinciding with the starting point of the diastolic pressure and ending at a final instant coinciding with the starting point of the subsequent diastolic pressure and comprising a dicrotic point, each heartbeat having a systolic phase comprised between the initial instant and the dicrotic point; and
   B. automatically analyze and discriminate the morphology of the sampled pressure P(t) signal for each heartbeat, determining the instant and pressure value of one or more characteristic points of the signal P(t) selected from the group comprising : - the initial instant; a point of systolic pressure corresponding to the absolute maximum of the pressure signal ; - a dicrotic point; and one or more resonance points, each of which occurs when a second derivative $d_2P/dt^2$ of the pressure signal P(t) has a relative maximum, wherein at least a characteristic point of the pressure signal P(t) belongs to the systolic phase of the heartbeat and is different from the initial instant;

C. for each heartbeat determining an energy efficiency value through the following sub-steps

C.1 determining a direct dynamic pressure wave impedance $Z_{d\text{-}D}(t)$ for each of said one or more characteristic points belonging to the systolic phase of the heartbeat into consideration with the exception of the initial instant, said direct dynamic pressure wave impedance $Z_{d\text{-}D}(t)$ being given by the ratio between a value of the pressure signal $P(t)$ in the characteristic point and the distance of the instant of the characteristic point from the initial instant and determining an impedance $Z_D$ of a direct pressure wave by adding with alternating signs the values of the direct dynamic pressure impedances $Z_{d\text{-}D}(t)$ ordered according to a direct temporal order starting from the initial instant up to the dicrotic instant, applying a positive sign to the first direct dynamic pressure impedance $Z_{d\text{-}D}(t)$ according to the direct temporal order;

C.2 for each of said one or more characteristic points determining a dynamic reflected impedance $Z_{d\text{-}R}(t)$ that is given by the ratio between the pressure at the characteristic point and the distance of the respective instant from the final instant, and determining the value of an impedance $Z_R$ of reflected waves of pressure by adding the dynamic reflected impedances with alternating signs of the second set of points thus determined, sorted according to their reverse temporal order starting from the final instant to the initial instant, giving a positive sign to the first dynamic reflected impedance $Z_{d\text{-}R}(t)$ according to the reverse temporal order;

C.3 determining the said energy efficiency as the ratio between the impedance $Z_D$ of the direct wave pressure and the impedance $Z_R$ of the reflected waves: $RES=Z_D/Z_R$.

2.  Method according to claim 1, **characterized in that** it further comprises the following steps:

D. check, in relation to said energy efficiency determined in step C, if a first derivative $dP/dT$ of the pressure signal $P(t)$ is lower than a first maximum threshold value $T_d$ throughout the entire heartbeat into consideration and if the second derivative $d_2P/dT^2$ is lower than a second maximum threshold value $T_{d2}$ throughout the entire heartbeat into consideration and in the negative case execute the step E, otherwise, execute the step F;

E. select a cutoff frequency of a low-pass filter on the basis of the energy efficiency determined in step C, of the first derivative $dP/dT$ and the second derivative $d_2P/dT^2$, and apply said low-pass filter to the pressure signal $P(t)$, obtaining a new sampled pressure signal, and execute the previous steps starting from step B;

F. providing, as exit signal, the signal $P(t)$ on which the step B has been executed for the last time.

3.  Method according to claim 1 **characterized in that** said one or more resonance points are determined in step B by the following sub-steps:

B.2 determining the total number $N_{dP\text{-}max}$ of points of relative maximum of the first derivative $dP/dt$ of the pressure signal (sampled) in the range of a single heartbeat;

B.3 determining the points of relative maximum of the second derivative $d^2P/dt^2$ of the pressure signal in the range of a single heartbeat, and

B.4 determining the $N_{dP\text{-}max}$ points of relative maximum of the second derivative $d^2P/dt^2$ having the highest values and determining the $N_{dP\text{-}max}$ time instants $t_{d2P\_max}(i)$ in which they occur, considering the points of the pressure signal in said $N_{dP\text{-}max}$ time instants $t_{d2Pmax}(i)$ as resonance points.

4.  Method according to claims 1 or 3 **characterized in that**, in the step B, the following characteristic points of the pressure signal $P(t)$ are determined:

- the initial instant,
- the point of systolic pressure,
- the dicrotic point, and
- one or more resonance points.

5.  Method according to anyone of claims 2-4 **characterized in that** said first maximum threshold value $T_d$ and second maximum threshold value $T_{d2}$ depend on the energy efficiency determined in step C.

6.  Method according to anyone of claims 2-5 **characterized in that**, in said step D, it is checked if said energy efficiency determined in step C belongs to one of three or more, preferably four, adjacent variability intervals, the first maximum threshold value $T_d$ and second maximum threshold value $T_{d2}$ preferably depending on the interval to which the energy efficiency determined in step C belongs.

7. Method according to claim 6 **characterized in that**, in said step E, the cutoff frequency is selected as follows:

- RES values are discriminated in three or more, preferably four, adjacent intervals of variability ;
- for each of said three or more adjacent intervals of variability of the energy efficiency determined in step C, the values of first derivative dP/dt of the pressure signal in the whole heartbeat are discriminated in three or more, preferably six, adjacent intervals of variability;
- ,and, for each of said variability intervals of the first derivative dP/dt of the pressure signal P(t) in the whole heartbeat, the values of the second derivative $d_2P/dt^2$ of the pressure signal are discriminated into three or more, preferably four, not superimposed variability intervals to which corresponds a respective value of the said cut-off frequency.

8. Method according to anyone of claims 2-7 **characterized in that** said cutoff frequency increases as the first derivative dP/dT of the pressure signal P(t) increases, with the energy efficiency and the second derivative $d_2P/dT^2$ of the pressure signal P(t) being constant.

9. Method according to anyone of claims 2-8 **characterized in that** said cutoff frequency decreases as the second derivative $d_2P/dT^2$ of the pressure signal P(t) increases, with the energy efficiency and the first derivative dP/dT of the pressure signal P(t) being constant.

10. Method according to anyone of claims 2-9 **characterized in that** said cutoff frequency is variable between 0.5 Hz and 10Hz, preferably between 2 Hz and 80 Hz, more preferably between 3Hz and 60Hz.

11. Method according to anyone of claims 2-10 **characterized in that** in step F the pressure signal P(t) is shown on a display.

12. Method according to anyone of claims 1-11 **characterized in that** in step C the RES is shown on a display.

13. Automatic apparatus for processing blood pressure signal **characterized in that** it comprises processing means for executing the steps of the method according to anyone of claims 1-12.

14. Computer program, comprising coded means for executing, when operating in connection with processing means of an apparatus, the steps of the method according to anyone of claims 1-12.

15. Memory support readable by computer means, having a program stored in it, **characterized in that** the program is the computer program according to claim 14.


**Patentansprüche**

1. Verfahren für die automatische Verarbeitung von Blutdruck-Signalen, das zumindest die folgenden Schritte umfasst:

A. Abtasten eines detektierten Drucksignals P(t) für einen oder mehrere Herzschläge, wobei jeder Herzschlag bei einem Anfangszeitpunkt beginnt, der mit dem Startpunkt des diastolischen Drucks zusammenfällt, und bei einem Endzeitpunkt aufhört, der mit dem Anfangszeitpunkt des nachfolgenden diastolischen Drucks zusammenfällt und einen dikrotischen Punkt umfasst, wobei jeder Herzschlag eine systolische Phase aufweist, die zwischen dem Anfangszeitpunkt und dem dikrotischen Punkt liegt,
B. automatisches Analysieren und Diskriminieren der Morphologie des abgetasteten Drucksignals P(t) für jeden Herzschlag, wobei der Moment und der Druckwert von einem oder mehreren charakteristischen Punkten des Signals P(t) ermittelt werden, die aus der folgenden Gruppe ausgewählt sind: Anfangszeitpunkt, ein Punkt des systolischen Drucks der dem absoluten Maximum des Drucksignals entspricht, ein dikrotischer Punkt und einer oder mehrere Resonanzpunkte, von denen jeder dann auftritt, wenn eine zweite Ableitung $d_2P/dt^2$ des Drucksignals P(t) ein relatives Maximum besitzt, wobei zumindest ein charakteristischer Punkt des Drucksignals P(t) zur systolischen Phase des Herzschlages gehört und vom Anfangszeitpunkt verschieden ist, und
C. für jeden Herzschlag Ermitteln eines Energie-Effizienzwertes durch die folgenden Unterschritte:

C.1 Ermitteln einer direkten dynamischen Druckwellenimpedanz $Z_{d\_D}(t)$ für einen oder mehrere der besagten charakteristischen Punkte, die zur betrachteten systolischen Phase des Herzschlages mit Ausnahme des Anfangszeitpunktes gehören, wobei die besagte direkte dynamische Druckwellenimpedanz $Z_{d\_D}(t)$

durch das Verhältnis zwischen einem Wert des Drucksignals P(t) im charakteristischen Punkt und dem Abstand des Moments des charakteristischen Punktes vom Anfangszeitpunkt gegeben ist, und Ermitteln einer Impedanz $Z_D$ einer direkten Druckwelle durch Addieren mit wechselnden Vorzeichen der Werte der direkten dynamischen Druckimpedanz $Z_{d\_D}(t)$ nach einer direkten zeitlichen Ordnung geordnet beginnend vom Anfangszeitpunkt bis zum dikrotischen Moment, wobei der gemäß der direkten zeitlichen Reihenfolge ersten dynamischen Druckimpedanz $Z_{d\_D}(t)$ ein positives Vorzeichen zugeordnet wird,

C.2 Ermitteln einer dynamischen reflektierten Impedanz $Z_{d-R}(t)$ für einen oder mehrere der besagten charakteristischen Punkte, die durch das Verhältnis zwischen dem Druck an dem charakteristischen Punkt und dem Abstand des betreffenden Moments vom Endzeitpunkt gegeben ist, und Ermitteln des Wertes einer Impedanz $Z_R$ der reflektierten Druckwellen durch Addieren der dynamischen reflektierten Impedanzen mit alternierenden Vorzeichen des zweiten Satzes von derart ermittelten Punkten, sortiert nach ihrer umgekehrten zeitlichen Reihenfolge beginnend mit dem Endzeitpunkt bis zum Anfangszeitpunkt, wobei der gemäß der umgekehrten zeitlichen Reihenfolge ersten dynamischen reflektierten Impedanz $Z_{d-R}(t)$ ein positives Vorzeichen zugeordnet wird,

C.3 Ermitteln der Energieeffizienz als das Verhältnis zwischen der Impedanz $Z_D$ des direkten Wellendrucks und der Impedanz $Z_R$ der reflektierten Wellen: RES = $Z_D/Z_R$.

**2.** Verfahren nach Anspruch 1, **dadurch gekennzeichnet, dass** es weiterhin folgende Schritte umfasst:

D. Überprüfung, ob in Bezug auf die in Schritt C ermittelte Energieeffizienz eine erste Ableitung dP/dT des Drucksignals P(t) über den gesamten in Auswertung befindlichen Herzschlag hinweg kleiner ist als ein erster maximaler Schwellenwert $T_d$ und ob die zweite Ableitung $d_2P/dt^2$ den ganzen in Auswertung befindlichen Herzschlag hinweg kleiner ist als ein zweiter maximaler Schwellenwert $T_{d2}$ und, wenn dies nicht der Fall ist, Durchführung des Schrittes E, ansonsten Durchführung des Schrittes F,

E. Auswahl einer Grenzfrequenz eines Tiefpassfilters auf der Basis der in Schritt C ermittelten Energieeffizienz der ersten Ableitung dP/dT und der zweiten Ableitung $d_2P/dt^2$, und Anlegen des Tiefpassfilters an das Drucksignal P(t), wodurch ein neues abgetastetes Drucksignal erhalten wird, und Durchführen der vorausgehenden Schritte beginnend mit Schritt B,

F. Bereitstellen des Signals P(t), an dem der Schritt B zum letzten Mal durchgeführt worden ist, als Ausgangssignal.

**3.** Verfahren nach Anspruch 1, **dadurch gekennzeichnet, dass** ein oder mehrere Resonanzpunkte in Schritt B durch die folgenden Teilschritte:

B.2 Ermitteln der Gesamtzahl $N_{dP-max}$ von Punkten eines relativen Maximums der ersten Ableitung dP/dT des Drucksignals (abgetastet) im Bereich eines einzelnen Herzschlages,

B.3 Ermitteln der Punkte eines relativen Maximums der zweiten Ableitung $d_2P/dt^2$, des Drucksignals im Bereich eines einzelnen Herzschlages,

B.4 Ermitteln der $N_{dP-max}$-Punkte eines relativen Maximums der zweiten Ableitung $d_2P/dt^2$, welche die höchsten Werte aufweisen, und Ermitteln der $N_{dP-max}$-Zeitpunkte $t_{d2P\_max}(i)$, in denen sie auftreten, wobei die Punkte des Drucksignals zu diesen $N_{dP-max}$-Zeitpunkten $t_{d2P\_max}(i)$ als Resonanzpunkte betrachtet werden.

**4.** Verfahren nach Anspruch 1 oder 3, **dadurch gekennzeichnet, dass** im Schritt B die folgenden charakteristischen Punkte des Drucksignals P(t) ermittelt werden:

- der Zeitmoment,
- der Punkt des systolischen Drucks,
- der dikrotische Punkt und
- ein oder mehrere Resonanzpunkte.

**5.** Verfahren nach einem der Ansprüche 2 bis 4, **dadurch gekennzeichnet, dass** der erste maximale Schwellenwert $T_d$ und der zweite maximale Schwellenwert $T_{d2}$ von der in Schritt C ermittelten Energieeffizienz abhängen.

**6.** Verfahren nach einem der Ansprüche 2 bis 5, **dadurch gekennzeichnet, dass** in Schritt D geprüft wird, ob die im Schritt C ermittelte Energieeffizienz zu einem von drei oder mehr, vorzugsweise vier benachbarten Variabilitätsintervallen gehört, wobei der erste maximale Schwellenwert $T_d$ und der zweite maximale Schwellenwert $T_{d2}$ vorzugsweise von dem Intervall abhängen, zu dem die in Schritt C ermittelte Energieeffizienz gehört.

7. Verfahren nach Anspruch 6, **dadurch gekennzeichnet, dass** in Schritt E die Grenzfrequenz in folgender Weise ausgewählt wird:

- es werden RES-Werte in drei oder mehr, vorzugsweise in vier Variabilitätsintervallen ausgewählt,
- für jedes der drei oder mehr benachbarten Variabilitätsintervalle der in Schritt C ermittelten Energieeffizienz werden die Werte der ersten Ableitung dP/dT des Drucksignals im gesamten Herzschlag in drei oder mehr, vorzugsweise sechs benachbarten Variabilitätsintervallen diskriminiert, und
- für jedes der Variabilitätsintervalle der ersten Ableitung dP/dT des Drucksignals P(t) des gesamten Herzschlages werden die Werte der zweiten Ableitung $d_2P/dt^2$ des Drucksignals in drei oder mehr, vorzugsweise vier nicht überlagerte Variabilitätsintervalle diskriminiert, denen ein entsprechender Wert der Grenzfrequenz entspricht.

8. Verfahren nach einem der Ansprüche 2 bis 7, **dadurch gekennzeichnet, dass** die Grenzfrequenz anwächst, wenn die erste Ableitung dP/dT des Drucksignals P(t) anwächst, wobei die Energieeffizienz und die zweite Ableitung $d_2P/dt^2$ des Drucksignals P(t) konstant sind.

9. Verfahren nach einem der Ansprüche 2 bis 8 **dadurch gekennzeichnet, dass** die Grenzfrequenz abnimmt, wenn die zweite Ableitung $d_2P/dt^2$ des Drucksignals P(t) anwächst, wobei die Energieeffizienz und die erste Ableitung dP/dT des Drucksignals P(t) konstant sind.

10. Verfahren nach einem der Ansprüche 2 bis 9, **dadurch gekennzeichnet, dass** die Grenzfrequenz zwischen 0,5 Hz und 10 Hz, vorzugsweise zwischen 2 Hz und 80 Hz, noch mehr bevorzugt zwischen 3 Hz und 60 Hz variabel ist.

11. Verfahren nach einem der Ansprüche 2 bis 10, **dadurch gekennzeichnet, dass** im Schritt F das Drucksignal P(t) in eine Anzeigevorrichtung angezeigt wird.

12. Verfahren nach einem der Ansprüche 1 bis 11, **dadurch gekennzeichnet, dass** im Schritt C das RES in einer Anzeigeeinheit angezeigt wird.

13. Automatisch arbeitende Vorrichtung für die Verarbeitung eines Blutdruck-Signals, **dadurch gekennzeichnet, dass** sie Verarbeitungseinrichtungen zur Durchführung der Verfahrensschritte nach einem der Ansprüche 1 bis 12 umfasst.

14. Computerprogramm, das kodierte Einrichtungen zur Durchführung der Schritte des Verfahrens nach einem der Ansprüche 1 bis 12 umfasst, wenn diese in Verbindung mit Verarbeitungseinrichtungen einer Vorrichtung arbeiten.

15. Speichereinrichtung, die von Computereinrichtungen ausgelesen werden kann und in der ein Programm gespeichert ist, **dadurch gekennzeichnet, dass** das Programm das Computerprogramm nach Anspruch 14 ist.

**Revendications**

1. Procédé pour le traitement automatique des signaux de pression sanguine, comprenant au moins les étapes suivantes:

A. avoir à disposition un signal de pression détectée P(t) échantillonné à un ou plusieurs battements cardiaques, chaque battement cardiaque commençant à un instant initial qui coïncide avec le point de départ de la pression diastolique et se terminant à un instant final qui coïncide avec le point de départ de la pression diastolique subséquent et comprenant un point dicrotique, chaque battement cardiaque ayant une phase systolique comprise entre l'instant initial et le point dicrotique; et
B. analyser automatiquement et discriminer la morphologie du signal de la pression échantillonnée P (t) pour chacun battement cardiaque, déterminant l'instant e la valeur de la pression d'un ou plusieurs points caractéristiques du signal P (t) choisi dans le groupe comprenant: - l'instant initial; un point de pression systolique correspondent à le maximum absolu du signal de la pression; un point dicrotique; et un ou plusieurs points de résonance, dont chacun se produit quand une deuxième dérivé $d_2P/dt^2$ du signal de pression P(t) présente un maximum relatif, dans lequel au moins un point caractéristique du signal de pression P(t) appartient à la phase systolique du battement cardiaque et est différent de l'instant initial;
C. pour chaque battement cardiaque déterminer une valeur de l'efficacité énergétique à travers les sous-étapes suivantes :

C.1 déterminer une impédance d'onde de pression dynamique directe $Z_{d-D}(t)$ pour chacun desdits un ou plusieurs points caractéristiques appartenant à la phase systolique du battement cardiaque en considération, à l'exception de l'instant initial, ladite impédance d'onde de pression dynamique directe $Z_{d\_D}(t)$ étant donné par le rapport entre une valeur du signal de pression P(t) dans le point caractéristique et la distance de l'instant du point caractéristique à partir de l'instant initial et déterminer une impédance $Z_D$ d'une onde de pression directe en ajoutant avec une alternance de signe les valeurs des impédances de pression dynamique directe $Z_{d\_D}(t)$ ordonnées selon un ordre temporel directe à partir de l'instant initial jusqu'à l'instant dicrotique, avec l'application d'un signe positif pour la première impédance de pression directe dynamique $Z_{d\_D}(t)$ en fonction de l'ordre temporel directe;

C.2 pour chacun desdits un ou plusieurs points caractéristiques déterminer une impédance dynamique réfléchie $Z_{d\_R}(t)$ qui est donné par le rapport entre la pression au niveau du point caractéristique et la distance du respectif instant de l'instant final, et déterminer la valeur d'une d'impédance $Z_R$ d'ondes réfléchies de pression en ajoutant les impédances dynamiques réfléchies avec alternance de signes de la seconde série de points ainsi déterminés, arrangés en fonction de leur ordre temporel inverse à partir de l'instant final à l'instant initial, donnant un signe positif à la première impédance dynamique réfléchie $Z_{d\_R}(t)$ en fonction de l'ordre temporel inverse;

C.3 déterminer ladite efficacité énergétique comme le rapport entre l'impédance $Z_D$ de la pression de l'onde directe et l'impédance $Z_R$ des ondes réfléchies: RES = $Z_D/Z_R$.

2. Procédé selon la revendication 1, **caractérisé en ce qu'**il comprend en outre les étapes suivantes:

D. vérifier, par rapport à ladite efficacité énergétique déterminé à l'étape C, si une première dérivée dP/dT du signal de pression P(t) est inférieure à une première valeur maximale de seuil $T_d$ pendant toute la durée du battement cardiaque considéré et si la dérivée seconde $d_2p/dT^2$ est inférieure à une seconde valeur de seuil maximale $T_{d2}$ pendant toute la durée du battement cardiaque considéré et, dans le cas négatif exécuter l'étape E, sinon, exécuter l'étape F;

E. choisir une fréquence de coupure d'un filtre passe-bas sur la base de l'efficacité de l'énergie déterminée à l'étape C, de la dérivée première dP/dT et de la dérivée seconde $d_2p/dT^2$, et appliquer ledit filtre passe-bas à le signal de pression P(t), obtenant un nouveau signal de pression échantillonnée, et exécuter les étapes précédentes à partir de l'étape B;

F. fournir, comme signal de sortie, le signal P (t) sur laquelle l'étape B a été exécutée pour la dernière fois.

3. Procédé selon la revendication 1, **caractérisé en ce que** lesdits un ou plusieurs points de résonance sont déterminées à l'étape B par les sous-étapes suivantes:

B.2 déterminer le nombre total $N_{dP-max}$ de points de maximum relatif de la première dérivée dP/dt du signal de pression (échantillonné) dans la plage d'un seul battement cardiaque;

B.3 déterminer les points de maximum relatif de la dérivée seconde $d^2P/dt^2$ du signal de pression dans la plage d'un seul battement cardiaque, et

B.4 déterminer les $N_{dP-max}$ points de maximum relatif de la dérivée seconde $d^2P/dt^2$ ayant les valeurs les plus élevées et déterminer les $N_{dP-max}$ instants de temps $t_{d2p\_max}(i)$ de temps dans lesquels ils se produisent, compte tenu des points de signal de pression dans lesdits $N_{dP\_max}$ instants de temps NDP-max instants $t_{d2p\_max}(i)$ en tant que points de résonance.

4. Procédé selon la revendication 1 ou 3, **caractérisé en ce que**, dans l'étape B, les points caractéristiques suivants du signal de pression P(t) sont déterminés:

- le instant initial,
- le point de la pression systolique,
- le point dicrotique, et
- un ou plusieurs points de résonance.

5. Procédé selon l'une quelconque des revendications 2-4, **caractérisé en ce que** ledit premier valeur maximale de seuil $T_d$ et seconde valeur maximale de seuil $T_{d2}$ dépendent de l'efficacité énergétique déterminé à l'étape C.

6. Procédé selon l'une quelconque des revendications 2-5, **caractérisé en ce que**, dans ladite étape D, on vérifie si ladite efficacité énergétique déterminé à l'étape C est appartient à trois ou plus, de préférence quatre, intervalles de variabilité adjacentes, la première valeur maximal de seuil $T_d$ et seconde valeur de seuil maximale $T_{d2}$ de préférence dépendent de l'intervalle auquel appartient l'efficacité énergétique déterminé à l'étape C.

7. Procédé selon la revendication 6, **caractérisé en ce que**, dans ladite étape E, la fréquence de coupure est sélectionné comme suit:

   - les valeurs RES sont discriminés en trois ou plusieurs, de préférence quatre, des intervalles adjacents de variabilité;
   - pour chacun desdits trois ou plusieurs intervalles adjacents de la variabilité de l'efficacité de l'énergie déterminée à l'étape C, les valeurs de la première dérivée dP/dt du signal de pression dans l'entière ensemble du battement cardiaque sont discriminés en trois ou plus, de préférence six, des intervalles adjacents de variabilité;
   - et, pour chacun desdits intervalles de variabilité de la première dérivée dP/dt du signal de pression P (t) dans l'entière ensemble du battement cardiaque, les valeurs de la dérivée seconde $d_2P/dt^2$ du signal de pression sont discriminés en trois ou plus, de préférence quatre, intervalles de variabilité non superposent pas qui correspond à une valeur respective de ladite fréquence de coupure.

8. Procédé selon l'une quelconque des revendications 2-7, **caractérisé en ce que** ladite fréquence de coupure augmente à mesure que la première dérivée dP/dT du signal de pression P(t) augmente, avec l'efficacité énergétique et de la dérivée seconde $d_2P/dT^2$ de le signal de pression P (t) étant constante.

9. Procédé selon l'une quelconque des revendications 2-8, **caractérisé en ce que** ladite fréquence de coupure diminue lorsque la dérivée seconde $d_2P/dT^2$ du signal de pression P (t) augmente, avec l'efficacité énergétique et la dérivée première dP/dT du signal de pression P(t) étant constante.

10. Procédé selon l'une quelconque des revendications 2-9, **caractérisé en ce que** ladite fréquence de coupure est variable entre 0,5 Hz et 10 Hz, de préférence entre 2Hz et 80 Hz, de préférence entre 3 Hz plus et 60 Hz.

11. Procédé selon l'une quelconque des revendications 2-10, **caractérisé en ce que** dans l'étape F le signal de pression P (t) est représentée sur un écran.

12. Procédé selon l'une quelconque des revendications 1-11, **caractérisé en ce que** dans l'étape C, le RES est représentée sur un écran.

13. Appareil automatique pour le signal de tension artérielle de traitement **caractérisé en ce qu'**il comprend des moyens de traitement pour exécuter les étapes du procédé selon l'une quelconque des revendications 1-12.

14. Programme d'ordinateur, comprenant des moyens codés pour exécuter, lors de l'utilisation en liaison avec des moyens de traitement d'un dispositif, les étapes du procédé selon l'une quelconque des revendications 1-12.

15. Support de mémoire lisible par des moyens informatiques, ayant un programme mémorisé dedans, **caractérisé en ce que** le programme est le programme d'ordinateur selon la revendication 14.

FIG.1

(a)

(b)

FIG.2

FIG.3

FIG.4

**FIG.5**

**FIG.6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004084088 A

### Non-patent literature cited in the description

- **RM GARDNER.** Direct Blood Pressure Measurement - Dynamic Response Requirements. *Anesthesiology,* March 1981, vol. 54 (3), 227-236